Europäisches Patentamt

(19) European Patent Office

Office européen des brevets

(11) Publication number : **0 420 056 B1**

(12) **EUROPEAN PATENT SPECIFICATION**

(45) Date of publication of patent specification :
**12.01.94 Bulletin 94/02**

(51) Int. Cl.[5] : **A61K 9/02**, A61K 47/12, A61K 47/14

(21) Application number : **90118160.2**

(22) Date of filing : **21.09.90**

(54) **Use of fatty acids, fatty acid salts and fatty acid esters of mono-, di- or trihydroxy alkanes.**

(30) Priority : **27.09.89 EP 89730203**

(43) Date of publication of application :
**03.04.91 Bulletin 91/14**

(45) Publication of the grant of the patent :
**12.01.94 Bulletin 94/02**

(84) Designated Contracting States :
**BE DE DK ES FR IT NL**

(56) References cited :
**FR-A- 992 143**
**FR-A- 2 455 897**
**GB-A- 843 886**

(56) References cited :
**CHEMICAL ABSTRACTS, vol. 102, 1985, page
363, abstract no. 84447m, Columbus, Ohio, US;
& RO-A-83 314 (SUCIU et al.) 28-02-1984
DERWENT FILE SUPPLIER WPIL,
AN=89-118273 [16], Derwent Publications Ltd,
London, GB; & JP-A-1 063 512 (TAISHO PHAR-
MACEUT.)**

(73) Proprietor : **UNIVERSITEIT GENT
Sint-Pietersnieuwstraat 25
B-9000 Gent (BE)**

(72) Inventor : **De Muynck, Christian D.E.
Bremstraat 5
B-9050 Evergem (BE)**
Inventor : **Meert, Dirk M.
Vierkeerstraat 5
B-8752 Bavikhove (BE)**

## Description

BACKGROUND OF THE INVENTION

The invention relates to the use of fatty acids and/or fatty acid salts and/or fatty acid esters of mono-, di- or trihydroxy alkanes for the manufacture of fat-based suppositories.

Suppositories are solid dosage forms which can be inserted via rectum or vagina. They can be used either for the effect of the suppository base itself (e.g. laxatives), or for local effect (especially vaginal) or for a systemic effect resulting from drug release and absorption into the circulating system.

Two categories of suppository bases are on the market, fatty bases and water-soluble or water-dispersible bases which all meet the following requirements: they remain solid at room temperature, but melt, dissolve or disperse after insertion into the body, so that the drug contained in the base will be released.

Until 1940, suppository bases were almost exclusively made from cocoa butter (isolated from Theobroma cocoa seed). Cocoa butter, a natural product, is a mixture of triglycerides of saturated and unsaturated fatty acids which can be manipulated in solid form at room temperature and which melts completely at body temperature.

However, cocoa butter has a number of disadvantages such as variable quality, polymorphic behaviour which requires tempering during manufacture, chemical instability (literature claims that it becomes rancid, Coben L.J and Lieberman H.A., The Theory and Practice of Industrial Pharmacy, chapter 19, Suppositories, page 575) and variable price.

Therefore, different cocoa butter substitutes (CBS) are on the market and in use as suppository bases. Four categories are to be distinguished:
- interesterified fully hydrogenated palm kernel oil;
- fully hydrogenated palm kernel stearin;
- mid fractions of hydrogenated vegetable oils which are rich in trans fatty acids; and
- semi-synthetic glycerides, of which the production entails hydrolysis of natural vegetable oils, hydrogenation, distillation and finally re-esterification of particular fractions with glycerol under controlled conditions, to obtain a mixture of mono-, di and triglycerides with appropriate characteristics.

Non-lauric glycerides contain few or approximately no lauric (C12) acids, whereas lauric glycerides contain a considerable amount of lauric acids. Lauric semi-synthetic glycerides are commonly used suppository fat bases because their characteristics such as solid fat content can easily be controlled by appropriate choice of reagents. The main difficulty in preparing a cocoa butter substitute is to obtain a product which melts completely near body temperature and which remains sufficiently solid at about 30°C. Consequently, suppository fat bases tend to contain triglycerides which as pure compounds fall within a narrow melting point range and thus exhibit a sharp decrease in solid fat content. Trade names of commercially available fat bases are e.g. Suppocire (Etablissements Gattefosse, St. Priest), Novata (Henkel International, Düsseldorf) and Witepsol (Hüls Troisdorf AG).

In contrast to the fat bases glycerol-gelatine bases and the macrogol bases are water-soluble. The macrogol bases are mixtures of polyethyleneglycols (PEG, known as Carbowax and Polyglycol on the U.S. market) with different molecular weights, which disperse in the rectal fluid. Because there is only a small amount of rectal fluid, polyethyleneglycol draws in water by osmosis from the rectal mucosa. This leads to dehydration of the rectal mucosa, and irritation is an inevitable consequence (Bevernage K.B.M. and Prof. Polderman J., Farmaceutische Lessen, Zetpillen, page 433).

The literature reveals that the cocoa butter suppositories do not irritate the rectal mucosa, as do polyethyleneglycol suppositories (Introduction to Pharmaceutical Dosage Forms, chapter 14, Suppositories and Other Rectal, Vaginal, and Urethral Preparations, pages 331 and 332).

Also other bases, for example commonly used cocoa butter substitutes, are supposed not to irritate, and if irritation appears, this is always related to the drug, contained in the suppository base (Rectal Bleeding and Indomethacin, Brit. Med. J., 06/04/68, page 52; Lesions solitaires du rectum dues a des suppositoires associant acide acetylsalicylique et paracetamol, Lanthier P., Detry R., Debongnie J.C., Mahieu P., Vanheuverzwyn R., Gastroenterol. Clin. Biol., 1987, 11, 250-253; Anorectal Ergotism: Another Cause of Solitary Rectal Ulcers, Eckhardt V.F., Kanzler G., Remmele W., Gastroenterology, 1986, 92, page 1123-1127; Bioavailability and Tolerance Studies on Acetylsalicylic Acid Suppositories, Solvell L., Acta Pharm. Suec., 12, 491-498, 1975). It concerns drugs either for long-term application (indomethacin for rheumatoid arthritis, aminophilline for bronchospasms, ...) or of which abuse frequently appears (sedatives e.g. paracetamol, ASA, or drugs for treatment of migraine e.g. ergominetartrate).

However, contrary to the above assumption it has now been found that the fat bases themselves already cause rectal irritation and that therefore the irritation is not necessarily due to the drug. This is demonstrated by the following irritation studies according to which all suppository bases, including cocoa butter, are found to be irritating.

The following products were investigated:
- Polyethyleneglycol: a mixture of PEG 1540 (70 parts ) and PEG 6000 (30 parts); Dow Chemical;
- Witepsol H15 (1) (containing 2 % Cetomacrogol 1000);
  Hüls Troisdorf AG,
  Germany (Cetomacrogol 1000 = PEG 1000 monocetylic ether);
- Novata B (2); Henkel International, Düsseldorf, Germany;
- Suppocire AM (3) and Suppocire AP; Etablissements Gattefosse, St. Priest, France
- Cocoa butter (4)
- A non - Lauric CBS (5): Triglycerides of saturated and unsaturated fatty acids, namely a fraction of a partially hydrogenated mixture of soy bean oil and a palm oil fraction, developed by N.V. Vandemoortele Izegem, used as a confectionery fat and sold under the trademark Mesuro PS.

Samples of the products were analysed by gas liquid chromatography for fatty acid composition and carbon number. The obtained glyceride compositons are summarized in Table 1a. The fatty acid compositions are summarized in Table 1b. The acid values, hydroxyl values, iodine values, melting points and saponification values (Handbook of Pharmaceutical recipients, Semi-Synthetic Glycerides, page 315-317) are summarized in Table 1c.

## Table 1a:

|       | (1)    | (2)    | (3)    | (4)    | (5)   |
|-------|--------|--------|--------|--------|-------|
| Mono  | 0.3%   | 1.4%   | ≤0.5%  | ≤0.2%  | -     |
| Di    | 10.7%  | 18.9%  | ≤3.0%  | ≤4.0%  | 2.9%  |
| Tri   | 89.0%  | 79.7%  | ≥96.5% | ≥95.8% | 97.1% |

## Table 1b:

|        | (1)  | (2)  | (3)  | (4)    | (5)  |
|--------|------|------|------|--------|------|
| 8:0    | 0.1  | -    | 4.2  | -      | -    |
| 10:0   | 1.2  | 0.3  | 3.7  | -      | -    |
| 12:0   | 54.3 | 53.5 | 48.4 | ≤0.25  | 0.1  |
| 14:0   | 19.7 | 20.1 | 15.1 | ≤0.25  | 0.3  |
| 16:0   | 12.7 | 11.8 | 8.3  | 26.0   | 17.5 |
| 18:0   | 12.8 | 13.9 | 19.4 | 35.0   | 6.5  |
| 18:1   | 0.1  | -    | 0.2  | 34.0   | 67.5 |
| 18:2   | -    | -    | -    | 3.0    | 6.2  |
| 18:3   | -    | -    | -    | ≤0.25  | -    |
| 20:0   | 0.1  | 0.2  | 0.2  | 1.0    | 0.7  |
| 20:1   | -    | -    | -    | -      | 0.3  |
| 22:0   | -    | -    | -    | ≤0.25  | 0.4  |

3

Table 1c:

|  | (1) | (2) | (3) |
|---|---|---|---|
| AV | $\leqslant 0.2$ | $\leqslant 0.3$ | $\leqslant 0.2$ |
| HV | $\leqslant 13$ | 20-30 | $\leqslant 6$ |
| IV | $\leqslant 3$ | $\leqslant 3$ | $\leqslant 2$ |
| MP | 33.5-35 | 33.5-35.5 | 35-36.5 |
| SV | 230-240 | 225-240 | 225-245 |

(AV = acid value; HV = hydroxyl value; IV = iodine value; MP = melting point; SV = saponification value)

The irritation studies were carried out on rabbits (breed: New Zealand, male, weight 2.3 - 2.7 kg at the beginning of the experiments). The recta of these animals are histologically analogous to those of humans, and in size to that of a baby of about six months. This justifies the use of suppositories of 1 g.

The rabbits were placed in metal cages, two per cage, and provided with water and food ad libitum. A suppository base of 1 g was administered three times a day (8h-16h-24h), for fourteen days. After administration of each suppository, the anus was kept shut for thirty minutes, by use of a clothespeg, to prevent the melted suppository base being expelled prematurely from the rectum, which would reduce time of contact between the rectal mucosa and the molten base. During this time the rabbits were immobilized, so that they could not remove the clothespeg.

The control group consisted of rabbits receiving no suppositories nor a clothespeg (incidence of natural occurence of rectal irritation), or receiving just a clothespeg (incidence of irritation as result of rectal obstruction). A third control group ("Placebo") received hard non-melting parrafin suppositories which could not be expelled within 30 minutes after insertion (incidence of irritation as result of application of the suppository). The recta and anuses of the killed rabbits were removed and immediately fixated in an aqueous 4% formaldehyde solution, and subsequently examined stereo-microscopically for surface injuries. Tissues of rectum-/anus preparations were cut longitudinally and embedded in paraffin. The tissues thus obtained were cut by use of a microtome into sections, and stained with haemotoxylin and eosin. On those sections histological examination was performed.

In examining the specimens, certain defined observations were recorded in accordance with a system of classification. Definitions and classifications are:

-hyperaemia: congestion of the mucosa, presence of red blood cells in the mucosa; - negative, + focal, ++ diffuse;

-oedema: oedematous mucosa and submucosa appear thicker than normal; the crypts fail to reach the muscularis mucosa and the space between them is decreased; the lamina propria appears empty or focally depleted; - negative, + focal (only in mucosa), ++ dispersed (in mucosa as well as in submucosa);

-inflammation: excessive number of inflammatory cells; -- negative, + focal inflammation, ++ diffuse, +++ heavy diffuse inflammation.

-erosion: local loss of surface epithelium; absent or present.

-ulceration: the surface epithelium is lost and replaced by a spray of fibrin, mucus and inflammatory cells microscopically visible; absent or present.

-regeneration: the mucosal layer is replaced by large undifferentiated epithelial cells with deeply stained cytoplasm and increased nucleocytoplasmic ratio; absent or present.

pseudopolyps: pseudopolyps or inflammatory polyps indicate a prior severe bout of mucosal ulceration with irregular healing; absent or present.

The results in percentages of the irritation studies are summarized in table 2.

4

Table 2:

| N= | | CON(-) 20 | CON(+) 20 | PLA 20 | MES 20 | CAC 20 | PEG 20 | SAM 20 | SAP 20 | WIT 20 | NOV 20 |
|---|---|---|---|---|---|---|---|---|---|---|---|
| HYPER. | NEG | 100 | 95 | 95 | 70 | 55 | 10 | 35 | 75 | 55 | 76 |
| | + | 0 | 5 | 5 | 20 | 40 | 25 | 55 | 25 | 40 | 21 |
| | ++ | 0 | 0 | 0 | 10 | 5 | 65 | 10 | 0 | 5 | 3 |
| OEDEM. | NEG | 95 | 95 | 95 | 50 | 75 | 65 | 25 | 45 | 80 | 82 |
| | + | 5 | 5 | 5 | 45 | 20 | 30 | 55 | 55 | 20 | 18 |
| | ++ | 0 | 0 | 0 | 5 | 5 | 5 | 20 | 0 | 0 | 0 |
| INFLAM. | NEG | 90 | 90 | 70 | 20 | 5 | 20 | 0 | 25 | 20 | 15 |
| | + | 10 | 10 | 30 | 30 | 50 | 35 | 75 | 60 | 25 | 12 |
| | ++ | 0 | 0 | 0 | 30 | 35 | 35 | 25 | 15 | 20 | 46 |
| | +++ | 0 | 0 | 0 | 20 | 10 | 10 | 0 | 0 | 35 | 27 |
| EROSION | NEG | 100 | 100 | 90 | 80 | 65 | 65 | 45 | 70 | 85 | 94 |
| | POS | 0 | 0 | 10 | 20 | 35 | 35 | 55 | 30 | 15 | 6 |
| ULCER. | NEG | 100 | 100 | 90 | 80 | 80 | 95 | 65 | 65 | 60 | 55 |
| | POS | 0 | 0 | 10 | 20 | 20 | 5 | 35 | 35 | 40 | 45 |
| REGENER. | NEG | 100 | 100 | 85 | 80 | 60 | 65 | 45 | 50 | 60 | 61 |
| | POS | 0 | 0 | 15 | 20 | 40 | 35 | 55 | 50 | 40 | 39 |
| PSEUDOP. | NEG | 100 | 100 | 95 | 50 | 50 | 65 | 50 | 65 | 50 | 79 |
| | POS | 0 | 0 | 5 | 50 | 50 | 35 | 50 | 35 | 50 | 21 |

CON(-) = no suppositories, no clothespeg; CON(+) = no suppositories, clothespeg used; PLA = Placebo (non-melting parrafin ); MES = Mesuro PS; CAC = Cacaobutter; PEG = Polyethyleneglycol; SAM = Suppocire AM; SAP = Suppocire AP; WIT = Witepsol H15; NOV = Novata; N = number of rabbits investigated.

Based upon stereomicroscopical observations, all fat bases examined were found to irritate the rectal mucosa. In the control group only two rabbits showed minimal hyperaemia, oedema and focal inflammation. That means that irritation is apparently not related to the presence of a clothespeg. Most sections of rabbits treated with suppository bases show not only hyperaemia of the lamina propria of the rectal mucosa, but also the so-called "Solitary Rectal Ulcer Syndrome" (SRUS). The presence of necrotic material (ulceration) and fibrous connective tissue and a massive infiltration of granulocytes in the lamina propria characterize the SRUS. The degree of hyperaemia of the lamina propria or the degree of irritation induced by the fatty bases varied according to the base used. The suppository bases can be listed in order of increasing irritation produced: cocoa butter, non-lauric CBS, Suppocire AP, Suppocire AM, Novata B, Witepsol H15, and the polyethyleneglycol base. For the latter a strongly pronounced hyperaemia of the lamina propria on the whole rectum surface as well as an enhanced presence of lymphocytes and granulocytes was observed.

The above reported experiments demonstrate that bases of triglycerides of mainly saturated fatty acids (expecially C12 - C14) cause rectal irritation when applied on a long-term basis. Cocoa butter and the non-lauric CBS having a much higher unsaturated fatty acid content, do not irritate as much as the other suppository bases which have been examined. The diglyceride concentration, which varies for the suppositories used (Witepsol H15: +/- 10 %; Novata B: +/- 20 %, Suppocire AM: 3 %), has apparently no influence on the irritation property of the fat bases.

OBJECT OF THE INVENTION

Given the irritation observed, it is the object of the invention to provide fat-based suppositories with significantly reduced rectal irritation.

DETAILED DESCRIPTION OF THE INVENTION

The invention is directed to the use of fatty acids and/or fatty acid salts and/or fatty acid esters of mono-, di- or trihydroxy alkanes or their derivatives with the exception of di- and triacyl glycerols for the manufacture of fat-based suppositories for reducing the rectal irritation caused by the fat base.

The present invention is based on the surprising finding that the above reported triglyceride base caused rectum irritation can be markebly supressed by the addition of soaps and/or saponifiable compounds to the fat base. In the present context, a soap is defined as any compound that yields fatty acids on acidification and a saponifiable compound is defined as any compound that yields soaps on reaction with alkaline. Examples of soaps are sodium palmitate, potassium oleate etc. and examples of fatty acid esters are monoacyl glycerols, diacyl glycerols, triglycerides, fatty acid esters (e.g. fatty acid methyl esters) etc. However, triglycerides have been found to cause rectal irritation and diacyl glycerols have been found not to reduce this irritation and are therefore excluded from the use according to the invention.

Various types of the above mentioned compounds have been examined for their effect on reducing triglyceride caused rectal irritation and have been found to vary in effectiveness. Fatty acids and fatty acid salts have only a moderate effect, whereas for example fatty acid methyl esters and monoglycerides were found to be more effective on a per weight basis. It has generally been observed that the effectiveness in reducing rectal irritation is related to the concentration of the compound added to the suppository fat base.

Why and how these compounds reduce fat induced rectal irritation is not clear. It may be that they act as a kind of emulsifier and thus inhibit direct contact between the triglycerides in the suppository and the rectum mucosa, but an inclusion of these compounds into the lipophilic rectum mucosa membrane that renders it less sensible to triglyceride caused irritation may be another explanation of the phenomena observed.

The fatty acids and/or fatty acid salts and/or fatty acid esters of mono-, di- or trihydroxy alkanes useful in the present invention may be blended with the suppository fat base at several possible stages in the suppository manufacturing process. In case these compounds are volatile (monoglycerides) and/or heat sensible they are preferably added after the fat base has been deodorized. They can be added either by the fat base manufacturers prior to packaging or by the suppository manufacturer during formulation. In both cases, the compounds are preferably added to the molten fat in order to ensure complete and uniform distribution.

No fast rules can be given for the amount and type of the above mentioned compounds to be used. They depend to a large extent on the degree of reduction of rectal irritation required which may be less for suppositories used only incidentically than for suppositories for chronic medication, on the active ingredient used and on the effect these compounds may have on the physical properties of the suppository.

Nevertheless the following weight percentages based on the suppository fat-base should be used (preferred ranges in brackets):

| | |
|---|---|
| fatty acids | 3 - 20 (10 - 15) |
| fatty acid methyl esters | 3 - 10 ( 5 - 8) |
| monoglycerides | 3 - 15 ( 5 - 10) |

With respect to the active ingredient, a marked improvement in its availability has been observed as a result of the incorporation of minor amounts of fatty acids and/or fatty acid salts and/or fatty acid esters of mono-, di- or trihydroxy alkanes or their derivatives into the suppository fat base in a number of instances (Rectal Therapy, Müller B.W., Physicochemical Parameters Affecting Chemical Stability and Bioavailability of Drugs in Suppository Bases, page 22). However, such an improvement does not follow automatically so that bioavailability has to be measured for each intended combination of compound and active ingredient. Such measurements preferably include in vivo tests.

Care must be taken not to include too much of the compounds useful according to the invention, so as not to unduly affect the physical properties of the final suppository. This might lead to suppositories that are unacceptable soft and sticky during handling. A melting point depression of the fat base by the addition of these compounds can, however, be counteracted by an adjustment in triglyceride composition of the fat base.

If fatty acids are used to reduce rectal irritation they are preferably distilled before being added to and mixed into the fat base and the same holds for fatty acid esters of lower alcohols. There is no need to use pure fatty acids (e.g. 100 % stearic acid or 100 % oleic acid) or fatty acid esters although the use of pure compounds may offer advantages in maintaining the physical properties of the final products. In practice, mixtures can be quite acceptable.

Monoglycerides are preferably distilled so that their monoglyceride content is 90 wt.% or more. This avoids the inclusion of substantial amounts of diglycerides into the final product and thus avoids the risk of affecting the physical properties of the suppository. Lower monoglyceride concentrations (e.g. 45 %) in the saponifiable compound to be added to the suppository fat base are often quite acceptable though.

Example

To six series of N rabbits each (N = number of rabbits investigated), the following suppositories were administered, as described above, three times a day, for fourteen days:

(A) The non-lauric CBS fat base described above with the addition of respectively:
- 7.5 % fatty acids (1)
- 9.0 % fatty acids (1)
- 1.0 % methyl ester of fatty acids (1)
- 9.0 % fatty acids and 1 % methyl ester of fatty acids (1)
- 5.0 % Dimodan LS (unsaturated monoglycerides produced by Grindsted Vaerket) (2);

(B) Witepsol H15, with the addition of
- 9.0 % fatty acids and
1.0 % methyl ester of fatty acids (1)

The fatty acid compositions of the additives are summarized in Table 3. The results of the irritation studies, which were carried out as described above, are summarized in Table 4 (with regard to the symbols used please compare the explanations given above in connection with Table 2).

Table 3:

| C | (1) | (2) |
|---|-----|-----|
| 8:0 | – | 0.1 |
| 10:0 | – | 0.1 |
| 12:0 | 0.1 | 1.1 |
| 14:0 | 0.3 | 0.8 |
| 15:0 | – | 0.1 |
| 16:0 | 17.5 | 10.2 |
| 16:1 | – | 0.1 |
| 18:0 | 6.5 | 11.5 |
| 18:1 | 67.5 | 15.0 |
| 18:2 | 6.2 | 59.2 |
| 20:0 | 0.7 | 0.6 |
| 20:1 | 0.3 | 0.5 |
| 22:0 | 0.4 | 0.6 |

Table 4:

| N= | | MES+D 20 | MES+F 4 | MES+FF 4 | MES++ 20 | WIT++ 5 | MES+M 5 |
|---|---|---|---|---|---|---|---|
| HYPER. | NEG | 90 | 50 | 50 | 80 | 0 | 100 |
| | + | 10 | 50 | 50 | 20 | 100 | 0 |
| | ++ | 0 | 0 | 0 | 0 | 0 | 0 |
| OEDEM. | NEG | 80 | 100 | 50 | 90 | 40 | 60 |
| | + | 20 | 0 | 50 | 10 | 60 | 40 |
| | ++ | 0 | 0 | 0 | 0 | 0 | 0 |
| INFLAM. | NEG | 30 | 25 | 0 | 55 | 40 | 60 |
| | + | 25 | 75 | 100 | 45 | 60 | 20 |
| | ++ | 15 | 0 | 0 | 0 | 0 | 20 |
| | +++ | 0 | 0 | 0 | 0 | 0 | 0 |
| EROSION | NEG | 85 | 50 | 50 | 90 | 60 | 80 |
| | POS | 15 | 50 | 50 | 10 | 40 | 20 |
| ULCER. | NEG | 85 | 100 | 100 | 100 | 100 | 100 |
| | POS | 15 | 0 | 0 | 0 | 0 | 0 |
| REGENER. | NEG | 85 | 100 | 100 | 95 | 100 | 100 |
| | POS | 15 | 0 | 0 | 5 | 0 | 0 |
| PSEUDOP. | NEG | 75 | 50 | 75 | 95 | 80 | 100 |
| | POS | 25 | 50 | 25 | 5 | 20 | 0 |

```
MES    = Mesuro PS
MES+D  = Mesuro PS + 5% Dimodan LS
MES+F  = Mesuro PS + 7.5% Fatty Acids
MES+FF = Mesuro PS + 9% Fatty Acids
MES++  = Mesuro PS + 9% Fatty Acids + 1% Methyl Ester of Fatty
Acids
WIT++  = Witepsol H15 + 9% Fatty Acids + 1% Methyl Ester of
Fatty Acids
MES+M  = Mesuro PS + 1% Methyl Ester of Fatty Acids
```

These experiments indicate that the irritation induced by fat bases without addition of the compounds useful according to the present invention is much more considerable than irritation, if any observed, induced by fat bases to which such compounds are added.

## Claims

1. Use of fatty acids and/or fatty acid salts and/or fatty acid esters of mono-, di- or trihydroxy alkanes with the exception of di- and triacyl glycerols for the manufacture of fat-based suppositories for reducing the rectal or vaginal irritation caused by the fat-base.

2. Use according to claim 1 wherein the fat-base of the suppository is a non-lauric cocoa butter substitute.

## Patentansprüche

1. Verwendung von Fettsäuren, Fettsäuresalzen und Fettsäureestern von Mono-, Di- oder Trihydroxyalkanen mit Ausnahme von Di- und Triacylglycerolen für die Herstellung von Suppositorien auf Fettgrundlage, um die durch die Fettgrundlage verursachte rektale bzw. vaginale Reizung zu vermindern.

2. Verwendung nach Anspruch 1, wobei die Fettgrundlage des Suppositoriums ein Kakaobutter-Erzats ohne laurinsäure ist.

## Revendications

1. Utilisation d'acides gras, de sels d'acides gras et d'esters d'acides gras et de mono-, di- ou trihydroxy alcanes à l'exception de di- et triacylglycerols pour la production de suppositoires à base gras, pour la réduction de l'irritation rectale et vaginale causée par la base gras.

2. Utilisation selon la revendication 1, ou l'excipient gras du suppositoire est un substituant non-laurique du beurre de cacao.